# EUROPÄISCHE PATENTSCHRIFT

(11) **EP 2 085 060 B1**
(45) Veröffentlichungstag und Bekanntmachung des Hinweises auf die Patenterteilung: **12.02.2014**
(21) Anmeldenummer: 09004477.7
(22) Anmeldetag: 13.12.2004
(51) Int. Cl.: D03D 13/00

(54) **Material zur Herstellung eines Stützverbandes**
Material for producing a support bandage
Matériau de fabrication d'un bandage support

(43) Veröffentlichungstag der Anmeldung: 05.08.2009
(62) Teilanmeldung aus: 04803811.1
(73) Patentinhaber: Lohmann & Rauscher GmbH & Co. KG, 56567 Neuwied (DE)
(72) Erfinder: Leuprecht, Helmut, 1130 Wien (AT)
(74) Vertreter: Seranski, Klaus

(56) Entgegenhaltungen:
- DE-U1- 20 011 824
- GB-A- 928 099

## Beschreibung

Die Erfindung betrifft ein Material zur Herstellung eines Stützverbandes mit einem mit einem härtbaren Kunststoff beschichteten und/oder imprägnierten und zumindest teilweise in Form eines zwei sich zwischen nebeneinander verlaufenden Schußfäden kreuzende Kettfäden aufweisenden Drehergewebes gebildeten textilen Träger.

Materialien mit einem mit einem härtbaren Kunststoff beschichteten und/oder imprägnierten textilen Träger werden als Ersatz für Gipsverbände eingesetzt, welche insbesondere aufgrund ihres hohen Gewichtes einen schlechten Tragekomfort bieten. Dagegen können die genannten Ersatzmaterialien in Form von Kunststoff-Festverbänden durch Wahl entsprechender Kunststoffe und Träger bei einem wesentlich geringeren Gewicht mit der gleichen Festigkeit bzw. Steifigkeit hergestellt werden, während gleichzeitig eine hinreichende Atmungsaktivität sichergestellt ist. Darüber hinaus können derartige Materialien auch zur Herstellung eines auch in ausgehärtetem Zustand noch geringfügig verformbaren Verbandes eingesetzt werden, wenn härtbare Kunststoffe mit einer entsprechenden Beschaffenheit eingesetzt werden.

Herkömmliche Kunststoff-Festverbände werden in Form eines bahnförmigem, bandagenartigen Materials geliefert und zur Herstellung des Stützverbandes um den zu stützenden Körperteil gewickelt, wobei die Aushärtung des Kunststoffes durch eine entsprechende Behandlung vor, während und/oder nach Anlegen des Verbandes veranlaßt wird. Je nach Kunststoff kann dabei eine UV-Härtung, eine Wärmehärtung oder eine Härtung mit Hilfe eines Lösungsmittels durchgeführt werden. Als besonders zweckmäßig hat sich dabei die Härtung mit Hilfe von kaltem Wasser als Lösungsmittel erwiesen, weil dadurch keine zu Hautirritationen führende Erwärmung des Kunststoffs und auch sonst keine Hautreaktion hervorgerufen wird.

Zur Anpassung des herkömmlichen Materials an die Körperform können in Längs- und Querrichtung dehnbare textile Träger eingesetzt werden, wobei die während des Anlegens des Verbandes erzeugte Verformung des Trägers durch die Aushärtung des Kunststoffes fixiert wird. Bei herkömmlichen Kunststoff-Festverbänden werden zum Erhalt der gewünschten Dehnbarkeit gewirkte Glasfaserträger eingesetzt, welche ihre Dehnbarkeit in Querrichtung, d. h. in Schußrichtung des Trägers, aus der Maschenstruktur des gewirkten Materials beziehen und deren Dehnbarkeit in Längsrichtung des bahnförmigen, bandagenartigen Materials, d. h. in Kettrichtung des gewirkten Trägers, auf die Steifigkeit der Glasfasern zurückzuführen ist, welche dazu führt, daß die einzelnen Maschen des gewirkten Trägers auch in der Kettrichtung noch verformbar bleiben.

Unter Verwendung derartiger textiler Träger hergestellte Materialien der vorstehend angegebenen Art sind beispielsweise in der US-PS-3,787,272 angegeben.

Beim Einsatz dieser Materialien wird es jedoch als problematisch angesehen, daß beim Zerschneiden daraus hergestellter Stützverbände Glasfaserstäube entstehen können, welche im Verdacht stehen, gesundheitsschädlich zu sein.

Angesichts dieser Probleme wurde bereits die Herstellung textiler Träger aus anderen Kunstfasern, wie etwa Polyestergarnen und Polyamidgarnen, vorgeschlagen. Diese Garne weisen jedoch eine wesentlich geringere Steifigkeit auf als Glasfasern, so daß unter Verwendung derartiger Garne hergestellte Materialien zur Herstellung von Stützverbänden üblicherweise nur in der Querrichtung, d.h. der Schußrichtung des gewirkten textilen Trägers, eine ausreichende Dehnbarkeit aufweisen, während sie in der Längsrichtung bzw. Kettrichtung nur noch eine geringfügige Verformbarkeit zeigen. Daher passen sich unter Verwendung derartiger Träger hergestellte Materialien nur schlecht an die Körperform an. Zur Lösung dieses Problems wird in der EP 0 356 446 B1 ein gewirkter Träger für ein Material zur Herstellung eines Stützverbandes vorgeschlagen, bei dem die in Längsrichtung verlaufenden Kettfäden aus einem wärmeschrumpfbaren Material bestehen. Der in dieser Schrift beschriebene Träger wird zunächst aus im allgemeinen als Multifilamentgarne vorliegenden Garnen gewirkt und dann einer Wärmebehandlung unterzogen. Diese Wärmebehandlung führt zu einer Schrumpfung der in Längsrichtung des Trägers verlaufenden Kettfäden, welche diesen Kettfäden und damit auch dem Träger als Ganzem eine Strukturveränderung verleiht, die wiederum eine Dehnung der Kettfäden und damit auch des gesamten Trägers in Längsrichtung ermöglicht.

Wenngleich mit derartigen Trägern eine zufriedenstellende Anpassung des daraus hergestellten Verbandmaterials an die Körperform erreicht werden kann, ist die Herstellung dieser Träger problematisch.

Angesichts dieser Probleme im Stand der Technik wurde in der DE 200 11 824 vorgeschlagen, einen Stützverbandträger der eingangs beschriebenen Art einzusetzen. Dabei können durch Einsatz eines im Vergleich zu gewirkten Strukturen einfachen Drehergewebes die angesprochenen Herstellungsprobleme gelöst und gleichzeitig eine zufriedenstellende Formstabilität des Trägermaterials erreicht werden. Dabei wird die Herstellung der bekannten Materialien mit einem Träger in Form eines Drehergewebes dadurch vereinfacht, daß einer der sich kreuzenden Kettfäden unterhalb der Schußfäden verläuft und der andere der sich kreuzenden Kettfäden oberhalb der Schußfäden angeordnet ist, wobei der unterhalb der Schußfäden verlaufende Kettfaden an den Kreuzungsstellen oberhalb des oberhalb der Schußfäden verlaufenden Kettfadens angeordnet ist.

Wenngleich derartige Materialien einfach herstellbar sind, eine zufriedenstellende Formstabilität gewährleisten und damit auch die zur Anpassung an die Körperform gewünschte Dehnbarkeit sichergestellt werden kann, hat es sich gezeigt, dass die Langzeitstabilität dieser Materialien noch zu wünschen übrig läßt.

Die Schrift GB-A-928 099 offenbart ein mit einem härtbaren Formgips beschichtetes Drehergewebe, wobei das Drehergewebe hochfeste Glasfasern aufweist.

Angesichts dieser Problem im Stand der Technik liegt der Erfindung die Aufgabe zugrunde, eine Weiterbildung der bekannten Materialien anzugeben, welche auch bei längerem Einsatz stabil bleibt.

Bei Einsatz herkömmlicher Materialien der eingangs beschriebenen Art wird in einigen Fällen eine Einschnürung unter Zugbelastung und/oder die Ausbildung wulstartiger Falten beobachtet. Dabei können sich diese beobachteten wulstartigen Falten bei einem Unterschenkelverband ringförmig um die Fessel und auf dem Vorfuß ausbilden. Die Falten laufen dabei an den genannten Stellen parallel zur Kettrichtung des Trägermaterials.

Im Hinblick auf diese Probleme wird erfindungsgemäß vorgeschlagen, bei einem Material mit einem Träger in Form eines Drehergewebes mindestens einen Schußfaden des Drehergewebes zumindest abschnittweise aus einem hochfesten Garn, wie etwa einem Polyestergarn, auszuführen.

Diese Lösung geht auf die Erkenntnis zurück, daß die beobachtete Ausbildung von Einschnürungen und wulstartigen Falten auf eine zu geringe Querstabilität des herkömmlichen Trägermaterials hervorgerufen wird. Dieser Mangel kann gemäß dem gerade erläuterten Gesichtspunkt der Erfindung durch den Einsatz eines hochfesten Schußfadens behoben werden, weil dieser hochfeste Schußfaden, beispielsweise in Form eines Polyestergarns, dem Trägermaterial eine erhöhte Quersteifigkeit verleiht.

Das Gewebe des erfindungsgemäßen Materials enthält Kettfäden aus einem Elastomermaterial.

Zum Erhalt der gewünschten Dehnbarkeit können wärmeschrumpfbare Kettfäden und/oder Schußfäden eingesetzt werden, welche vor und/oder nach dem Webvorgang einer Wärmebehandlung unterzogen werden, um so eine dehnbare Struktur zu erhalten. Das Gewebe des erfindungsgemäßen Materials wird Schußfäden aus einem Elastomermaterial auf, welche ihre Dehnbarkeit aus den Eigenschaften des Materials selbst und nicht aus der Fadenstruktur beziehen.

Im Hinblick auf eine ausreichende Stabilität des Materials unter gleichzeitiger Sicherstellung einer zufriedenstellenden Anpassung an die Körperform hat es sich als besonders günstig erwiesen, wenn das Material in Richtung der Kettfäden des Gewebes vor dem Härten des Kunststoffes eine Dehnbarkeit von mindestens 20 %, vorzugsweise mindestens 30 %, besonders bevorzugt etwa 60 bis 95 % aufweist, wobei das Material in Richtung der Kettfäden des Gewebes in ungedehntem Zustand zweckmäßigerweise eine Reißfestigkeit von mindestens 10 N/cm, vorzugsweise mindestens 20 N/cm, besonders bevorzugt etwa 30 bis 60 N/cm aufweist.

Unter den gleichen Gesichtspunkten (Anpassung an die Körperform und Materialstabilität) hat es sich als günstig erwiesen, wenn das Material in Richtung der Schußfäden des Gewebes vor dem Härten des Kunststoffes eine Dehnbarkeit von mindestens 30 %, vorzugsweise mindestens 40 %, besonders bevorzugt etwa 50 bis 100 % aufweist, wobei die Reißfestigkeit des Materials in Richtung der Schußfäden des Gewebes in ungedehntem Zustand zweckmäßigerweise 10 N/cm, vorzugsweise mindestens 20 N/cm, besonders bevorzugt etwa 30 bis 50 N/cm beträgt.

Ein vergleichsweise geringes Gewicht erfindungsgemäßer Materialien kann bei gleichzeitiger Sicherstellung einer ausreichend hohen Stabilität erhalten werden, wenn das Gewebe in ungedehntem Zustand ein Flächengewicht von etwa 50 bis 250 g/m², vorzugsweise etwa 100 bis 200 g/m², aufweist.

Die Schuß- und/oder Kettfäden des Gewebes des erfindungsgemäßen Materials können beispielsweise Naturfasern aufweisen. Besonders gute Eigenschaften werden jedoch erhalten, wenn die Schuß- und/oder Kettfäden des Gewebes zusätzlich oder alternativ Kunstfasern, wie etwa Polyamidfasern, Polyesterfasern, Polypropylenfasern od. dgl., aufweisen, weil derartige Kunstfasern eine besonders gute Einstellung der Dehnbarkeit, Reißfestigkeit und anderer mechanischer Eigenschaften ermöglichen. Dazu können die Schuß- und/oder Kettfäden zum Erhalt der Dehnbarkeit zumindest teilweise texturiert sein. Diese Texturierung kann durch Wärmebehandlung der Fasern oder durch Behandlung der Fasern mit einem Lösungsmittel erhalten werden.

Wie bei den bekannten Materialien gemäß DE 200 11 824, können die Drehergewebe erfindungsgemäßer Materialien unter Sicherstellung einer besonders hohen Stabilität besonders einfach hergestellt werden, wenn bei mindestens einem Paar sich kreuzender Kettfäden einer der sich kreuzenden Kettfäden unterhalb der Schußfäden verläuft, der andere der sich kreuzenden Kettfäden oberhalb der Schußfäden verläuft und der unterhalb der Schußfäden verlaufende Kettfaden an den Kreuzungsstellen oberhalb des oberhalb der Schußfäden verlaufenden Kettfadens angeordnet ist. Weiter wird eine besonders hohe Stabilität derartiger Träger in Form eines Drehergewebes erreicht, wenn mindestens zwei Schußfäden zwischen aufeinanderfolgenden Kreuzungsstellen der Kettfäden und/oder aufeinanderfolgenden Übergängen mindestens eines zusätzlichen Kettfadens angeordnet sind. In diesem Zusammenhang hat es sich im Hinblick auf den Erhalt der gewünschten erhöhten Quersteifigkeit unter gleichzeitiger Sicherstellung der ebenfalls gewünschten strukturellen Integrität als besonders günstig erwiesen, wenn mindestens einer der zwischen aufeinanderfolgenden Kreuzungsstellen und/oder aufeinanderfolgenden Übergängen angeordneten Schußfäden texturiert ist und ein anderer dieser Schußfäden aus einem hochfesten, vorzugsweisen glatten Garn, insbesondere Polyestergarn, gebildet ist. Dabei wird mit dem hochfesten Garn die gewünschte Quersteifigkeit sichergestellt, während mit dem texturierten Schußfaden die gewünschte strukturelle Integrität verbessert wird.

In dieser Beschreibung wird jedes im wesentlichen geradlinig und senkrecht zu den Kettfäden verlaufendes Fadensegment als "Schußfaden" bezeichnet. Zum Erhalt einer besonders guten Stabilität des Trägergewebes des erfindungsgemäßen Materials hat es sich als günstig erwiesen, wenn die einzelnen Schußfäden in Form von Fadensegmenten gebildet sind, von denen mindestens zwei über ein am Rand des Gewebes angeordnetes Verbindungssegment miteinander verbunden sind, wobei dieses Verbindungssegment einstückig mit den die Schußfäden bildenden Fadensegmenten vorliegen kann. Dabei kann die Bildung von Rissen oder andersartiger Beschädigungen am Rand des Gewebes vermieden werden, wenn mindestens ein zwei Schußfäden miteinander verbindendes Verbindungssegment mindestens einen, vorzugsweise mindestens zwei, vorhergehende Schußfäden umläuft, um so eine Kante des Gewebes aus miteinander verbundenen Fasersegmenten zu bilden.

Im Hinblick auf den Erhalt einer möglichst hohen Stabilität und Dehnbarkeit eines erfindungsgemäßen Materials bei gleichzeitiger Sicherstellung eines möglichst geringen Gewichtes hat es sich als günstig erwiesen, wenn das Gewebe in ungedehntem Zustand etwa 40 bis 80, vorzugsweise etwa 66 Kettfäden, d. h. bei Verwendung eines Drehergewebes etwa 20 bis 40, vorzugsweise etwa 33 Doppelfäden in Kettrichtung und/oder etwa 80 bis 150, vorzugsweise etwa 132 Schußfäden, d.h. 40 bis 75, vorzugsweise etwa 66 Doppelschußfäden bei Verwendung des vorstehend beschriebenen Drehergewebes auf 10 cm der Gewebelänge bzw. -breite aufweist.

Wie eingangs bereits erläutert, kann es sich bei dem härtbaren Kunststoff um einen wärmehärtbaren oder UV-härtbaren Kunststoff handeln. Allerdings wird auch bei der Herstellung eines erfindungsgemäßen Materials vorzugsweise ein mit einem Lösungsmittel, insbesondere kaltem Wasser, härtbarer Kunststoff eingesetzt. Im Hinblick auf den Erhalt einer möglichst hohen Hautverträglichkeit bei gleichzeitiger Sicherstellung der gewünschten mechanischen Eigenschaften hat sich die Verwendung eines Kunststoffes, der ein feuchtigkeitshärtendes Polyurethanprepolymer umfaßt, als besonders günstig erwiesen. Derartige Kunststoffe sind beispielsweise in der EP 0 093 780 B1 beschrieben, deren Offenbarungsgehalt hinsichtlich der darin beschriebenen aushärtbaren Kunststoffe hiermit durch ausdrückliche Inbezugnahme in diese Beschreibung aufgenommen wird. Zweckmäßigerweise beträgt der Anteil des Kunststoffes an dem erfindungsgemäßen Material etwa 30 bis 70 Gew.-%.

Wie der vorstehenden Erläuterung des erfindungsgemäßen Materials bereits zu entnehmen ist, zeichnet sich der zur Herstellung dieses Materials eingesetzte erfindungsgemäße Träger im wesentlichen dadurch aus, daß er eine gewebte Struktur aufweist, wobei der Träger in Form eines Drehergewebes mit zusätzlichen, keine anderen Kettfäden kreuzenden Kettfäden verwirklicht ist. Mit Hilfe eines derartigen Trägers kann das erfindungsgemäße Material mit einem erfindungsgemäßen Verfahren hergestellt werden, indem der Träger mit einem härtbaren Kunststoff beschichtet und/oder imprägniert wird, wobei der Träger vor und/oder nach der Beschichtung bzw. Imprägnierung mit dem Kunststoff einer Wärmebehandlung unterzogen werden kann, um dem Material so insgesamt die gewünschte Dehnbarkeit zu verleihen.

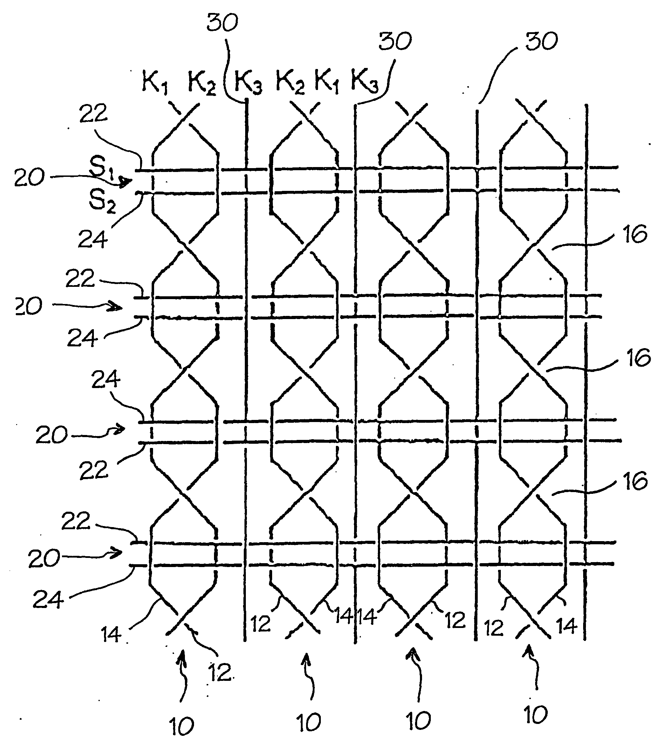

Nachstehend wird die Erfindung unter Bezugnahme auf die Zeichnung, auf die hinsichtlich aller erfindungswesentlichen und in der Beschreibung nicht näher herausgestellten Einzelheiten ausdrücklich verwiesen wird, erläutert. Die einzige Figur der Zeichnung zeigt eine schematische Darstellung einer Ausführungsform eines zur Herstellung eines erfindungsgemäßen Materials einsetzbaren erfindungsgemäßen Trägers.

Die Figur zeigt einen Ausschnitt eines erfindungsgemäßen Trägers für ein Material zur Herstellung eines Stützverbandes in Form eines Drehergewebes mit vier Kettfadenpaaren 10, mit sich kreuzenden Kettfäden 12 und 14 insgesamt vier Schußfadenpaaren 20 und drei zusätzlichen Schußfäden 30. Jedes der Kettfadenpaare 10 umfaßt einen unterhalb der Schußfadenpaare 20 verlaufenden Kettfaden 12 sowie einen oberhalb der Schußfadenpaare 20 verlaufenden Kettfaden 14. Dabei kreuzt der unterhalb der Schußfadenpaare 20 verlaufende Kettfaden 12 den oberhalb der Schußfadenpaare 20 verlaufenden Kettfaden 14 desselben Kettfadenpaares 10 an Kreuzungsstellen 16. An diesen Kreuzungsstellen 16 ist der unterhalb der Schußfadenpaare 20 verlaufende Kettfaden 12 oberhalb des oberhalb der Schußfadenpaare 20 verlaufenden Kettfadens 14 angeordnet. Zwischen aufeinanderfolgenden Kreuzungsstellen 16 der Kettfadenpaare 10 ist jeweils ein Schußfadenpaar 20 angeordnet, wobei die Kreuzungsstellen der einzelnen Kettfadenpaare 10 etwa auf einer parallel zu den Schußfadenpaaren 20 verlaufenden Geraden liegen. Durch die in der Zeichnung dargestellte Struktur eines Drehergewebes werden die Schußfadenpaare 20 fixiert, wodurch ein Verrutschen der einzelnen Fäden während der Beschichtung bzw. Imprägnierung mit dem härtbaren Kunststoff, wie etwa einem Polyurethanprepolymer, verhindert wird.

Jedes der Schußfadenpaare 20 enthält einen glatten Schußfaden 22 aus einem hochfesten Polyestergarn und einen texturierten Schußfaden 24, der ebenfalls aus einem Polyestergarn gebildet ist.

Zwischen den Kettfadenpaaren 10 sind zusätzliche, keine anderen Kettfäden kreuzende Kettfäden 30 angeordnet, welche abschnittweise oberhalb der Schußfäden und abschnittweise unterhalb der Schußfäden verlaufen. Dabei erfolgt der Übergang zwischen den oberhalb der Schußfäden verlaufenden Abschnitten und den unterhalb der Schußfäden verlaufenden Abschnitten zwischen denjenigen Schußfäden, zwischen denen sich auch die Kettfäden 12 und 14 der Kettfadenpaare 10 kreuzen. In der Zeichnung ist erkennbar, daß von zwei auf einander entgegengesetzten Seiten eines Paars sich kreuzender Kettfäden 12 und 14 angeordneten zusätzlichen Kettfäden 30 ein zusätzlicher Kettfaden 30 im Bereich der Schußfäden auf der dem anderen zusätzlichen Kettfaden 30 abgewandten Seite dieser Schußfäden 22, 24 angeordnet ist.

Die Erfindung ist nicht auf den Einsatz der anhand der Zeichnung erläuterten Drehergewebe beschränkt. Vielmehr können zur Herstellung eines erfindungsgemäßen Trägers auch Fäden aus natürlichen Fasern, Polyamidfasern, Polypropylenfasern u. dgl. eingesetzt werden. Dabei kann die Dehnbarkeit des unter Verwendung von Polyamidfasern hergestellten Trägers in ausgerüstetem, d. h. mit dem Kunststoff versehenem, ungedehntem Zustand in Kettrichtung 80 bis 90 % betragen. Das bedeutet, daß das ungedehnte Gewebe in Kettrichtung von 100 auf 180 bis 190 % gedehnt werden kann. In Schußrichtung kann die Dehnbarkeit eines aus Polyamidfasern hergestellten Trägers in ausgerüstetem (ungedehntem) Zustand 120 bis 130 % betragen. Das heißt, daß dieses Gewebe von 100 auf 220 bis 230 % gedehnt werden kann. Dabei kann das Flächengewicht in gedehntem Zustand 50 bis 55 g/m² betragen. Bei Einsatz von Polyamidfasern zur Herstellung der Kett- und Schußfäden kann eine Kettfadenstärke im Bereich von 40 bis 60 tex, vorzugsweise etwa 46,8 tex, und eine Schußfadenstärke im Bereich von 20 bis 40, vorzugsweise etwa 31,2 tex, zum Einsatz kommen.

Ferner können im Rahmen der Erfindung auch neue Kunststoffe, insbesondere an den Einsatz mit Polyamid angepaßte Kunststoffe in Form eines feuchtigkeitshärtenden Polyurethanprepolymers zum Einsatz kommen.

## Patentansprüche

1. Material zur Herstellung eines Stützverbandes mit einem mit einem härtbaren Kunststoff beschichteten und/oder imprägnierten und zumindest teilweise in Form eines zwei sich zwischen nebeneinander verlaufenden Schußfäden (22, 24) kreuzende Kettfäden (12, 14) aufweisenden Drehergewebes gebildeten Träger, wobei das Drehergewebe mindestens einen ein hochfestes Garn, insbesondere Polyestergarn, aufweisenden Schußfaden (22) aufweist,
**dadurch gekennzeichnet, dass** das Gewebe Kettfäden aus einem Elastomermaterial aufweist.

2. Material nach Anspruch 1, **dadurch gekennzeichnet, dass** das Gewebe wärmeschrumpfbare Kettfäden und/oder Schußfäden aufweist.

3. Material nach einem der vorhergehenden Ansprüche, **dadurch gekennzeichnet, dass** das Material in Richtung der Kettfäden (22, 24, 30) des Gewebes vor dem Härten des Kunststoffes eine Dehnbarkeit von mindestens 20 %, vorzugsweise mindestens 30 %, besonders bevorzugt etwa 65 bis 95 % aufweist.

4. Material nach einem der vorhergehenden Ansprüche, **dadurch gekennzeichnet, dass** das Material in Richtung der Kettfäden (22, 24, 30) des Gewebes in ungedehntem Zustand eine Reißfestigkeit von mindestens 10 N/cm, vorzugsweise mindestens 20 N/cm, besonders bevorzugt etwa 30 bis 60 N/cm aufweist.

5. Material nach einem der vorhergehenden Ansprüche, **dadurch gekennzeichnet, dass** das Material in Richtung der Schußfäden (22, 24) des Gewebes vor dem Härten des Kunststoffes eine Dehnbarkeit von mindestens 30 %, vorzugsweise mindestens 40 %, besonders bevorzugt etwa 50 bis 100 % aufweist.

6. Material nach einem der vorhergehenden Ansprüche, **dadurch gekennzeichnet, dass** das Material in Richtung der Schußfäden (22, 24) des Gewebes in ungedehntem Zustand eine Reißfestigkeit von mindestens 10 N/cm, vorzugsweise 20 N/cm, besonders bevorzugt etwa 30 bis 50 N/cm aufweist.

7. Material nach einem der vorhergehenden Ansprüche, **dadurch gekennzeichnet, dass** das Gewebe in ungedehntem Zustand ein Flächengewicht von etwa 50 bis 250 g/cm², vorzugsweise etwa 100 bis 200 g/cm² aufweist.

8. Material nach einem der vorhergehenden Ansprüche, **dadurch gekennzeichnet, dass** die Schuß- und/oder Kettfäden (12, 14, 22, 24, 30) des Gewebes Naturfasern aufweisen.

9. Material nach einem der vorhergehenden Ansprüche, **dadurch gekennzeichnet, dass** die Schuß- und/oder Kettfäden (12, 14, 22, 24, 30) des Gewebes Kunstfasern, wie etwa Polyamidfasern, Polyesterfasern, Polypropylenfasern oder dgl., aufweisen.

10. Material nach einem der vorhergehenden Ansprüche, **dadurch gekennzeichnet, dass** die Schuß- und/oder Kettfäden (12, 14, 22, 24, 30) zum Erhalt der Dehnbarkeit zumindest teilweise texturiert sind.

11. Material nach einem der vorhergehenden Ansprüche, **dadurch gekennzeichnet, dass** bei mindestens einem Paar sich kreuzender Kettfäden einer der sich kreuzenden Kettfäden (12) unterhalb der Schußfäden (22, 24) verläuft, der andere der sich kreuzenden Kettfäden (14) oberhalb der Schußfäden (22, 24) verläuft und der unterhalb der Schußfäden (22, 24) verlaufende Kettfaden (12) an den Kreuzungsstellen (16) oberhalb des oberhalb der Schußfäden (22, 24) verlaufenden Kettfadens (14) angeordnet ist.

12. Material nach einem der vorhergehenden Ansprüche, **dadurch gekennzeichnet, dass** mindestens zwei Schußfäden (22, 24) zwischen aufeinanderfolgenden Kreuzungsstellen (16) der Kettfäden (12, 14) und/oder aufeinanderfolgenden Übergängen mindestens eines zusätzlichen Kettfadens angeordnet sind.

13. Material nach Anspruch 12, **dadurch gekennzeichnet, dass** einer der zwischen aufeinanderfolgenden Kreuzungsstellen (16) und/oder aufeinanderfolgenden Übergängen angeordneten Schußfäden (22, 24) texturiert ist und ein anderer dieser Schußfäden (22, 24) aus einem hochfesten, vorzugsweise glatten Garn, insbesondere Polyestergarn, gebildet ist.

14. Material nach einem der vorhergehenden Ansprüche, **dadurch gekennzeichnet, dass** die Schußfäden (22, 24) in Form von Fadensegmenten gebildet sind, von denen mindestens zwei über ein am Rand des Gewebes angeordnetes Verbindungssegment miteinander verbunden sind.

15. Material nach Anspruch 14, **dadurch gekennzeichnet, dass** mindestens ein zwei Schußfäden miteinander verbindendes Verbindungssegment mindestens ein, vorzugsweise mindestens zwei vorhergehende Schußfäden umläuft.

16. Material nach einem der vorhergehenden Ansprüche, **dadurch gekennzeichnet, dass** das Gewebe in ungedehntem Zustand etwa 40 bis 80, vorzugsweise 66 Kettfäden und/oder etwa 80 bis 150, vorzugsweise 132 Schußfäden auf 10 cm der Gewebelänge bzw. -breite aufweist.

17. Material nach einem der vorhergehenden Ansprüche, **dadurch gekennzeichnet, dass** der Kunststoff mit einem Lösungsmittel, insbesondere Wasser, härtbar ist.

18. Material nach Anspruch 17, **dadurch gekennzeichnet, dass** der Kunststoff ein feuchtigkeitshärtendes Polyurethanprepolymer aufweist.

19. Material nach einem der vorhergehenden Ansprüche, **dadurch gekennzeichnet, dass** der Anteil des Kunststoffes an dem Material etwa 30 bis 70 Gew.-% beträgt.

20. Verfahren zum Herstellen eines Materials nach einem der Ansprüche 1 bis 19, **dadurch gekennzeichnet, dass** ein zumindest teilweise in Form eines zwei sich zwischen nebeneinander verlaufenden Schußfäden kreuzende Kettfäden aufweisenden Drehergewebes gebildeter Träger, wobei das Drehergewebe mindestens einen ein hochfestes Garn aufweisenden Schußfaden aufweist und Kettfäden aus einem Elastomermaterial aufweist, mit einem härtbaren Kunststoff beschichtet und/oder imprägniert wird.

## Claims

1. A material for producing a support bandage comprising a carrier coated and/or impregnated with a curable synthetic and made at least partially in the form of a leno fabric having two warp threads (12, 14) intersecting between weft threads (22, 24) running next to one another, said leno fabric having at least one weft thread (22) having a high strength yarn, in particular a polyester yarn,
**characterised in that** the fabric has warp threads made of an elastomeric material.

2. The material according to Claim 1, **characterised in that** the fabric has heat-shrinkable warp threads and/or weft threads.

3. The material according to any of the preceding claims, **characterised in that** the material has an extensibility of at least 20 %, preferably at least 30 %, particularly preferably approximately 65 to 95 % in the direction of the warp threads (22, 24, 30) of the fabric prior to hardening of the synthetic.

4. The material according to any of the preceding claims, **characterised in that** the material has a tensile strength of at least 10 N/cm, preferably at least 20 N/cm, particularly preferably approximately 30 to 60 N/cm in the direction of the warp threads (22, 24, 30) of the fabric in the unextended state.

5. The material according to any of the preceding claims, **characterised in that** the material has an extensibility of at least 30 %, preferably at least 40 %, particularly preferably approximately 50 to 100 % in the direction of the weft threads (22, 24) of the fabric prior to hardening of the synthetic.

6. The material according to any of the preceding claims, **characterised in that** the material has a tensile strength of at least 10 N/cm, preferably 20 N/cm, particularly preferably approximately 30 to 50 N/cm in the direction of the weft threads (22, 24) of the fabric in the unextended state.

7. The material according to any of the preceding claims, **characterised in that** the fabric has a weight per unit area of approximately 50 to 250 g/cm², preferably approximately 100 to 200 g/cm², in the unextended state.

8. The material according to any of the preceding claims, **characterised in that** the weft and/or warp threads (12, 14, 22, 24, 30) of the fabric have natural fibres.

9. The material according to any of the preceding claims, **characterised in that** the weft and/or warp threads (12, 14, 22, 24, 30) of the fabric have synthetic fibres, such as for example polyamide fibres, polyester fibres, polypropylene fibres or the like.

10. The material according to any of the preceding claims, **characterised in that** the weft and/or warp threads (12, 14, 22, 24, 30) are at least partially texturised to maintain extensibility.

11. The material according to any of the preceding claims, **characterised in that** with at least one pair of intersecting warp threads one of the intersecting warp threads (12) runs beneath the weft threads (22, 24), the other of the intersecting warp threads (14) runs above the weft threads (22, 24) and the warp thread (12) running beneath the weft threads (22, 24) is located above the warp thread (14) running above the weft threads (22, 24) at the intersection points (16).

12. The material according to any of the preceding claims, **characterised in that** at least two weft threads (22, 24) are located between successive intersection points (16) of the warp threads (12, 14) and/or successive crossovers of at least one additional warp thread.

13. The material according to Claim 12, **characterised in that** one of the weft threads (22, 24) located between successive intersection points (16) and/or successive crossovers is texturised and another of these weft threads (22, 24) is formed from a high strength, preferably smooth yarn, in particular polyester yarn.

14. The material according to any of the preceding claims, **characterised in that** the weft threads (22, 24) are made in the form of thread segments at least two of which are connected to one another by means of a connecting segment located at the edge of the fabric.

15. The material according to Claim 14, **characterised in that** at least one connecting segment connecting two weft threads to one another wraps around at least one, preferably at least two, preceding weft thread(s).

16. The material according to any of the preceding claims, **characterised in that** the fabric has approximately 40 to 80, preferably 66 warp threads and/or approximately 80 to 150, preferably 132 weft threads per 10 cm length or width of the fabric in the unextended state.

17. The material according to any of the preceding claims, **characterised in that** the synthetic can be cured with a solvent, in particular water.

18. The material according to Claim 17, **characterised in that** the synthetic has a moisture-hardening polyurethane prepolymer.

19. The material according to any of the preceding claims, **characterised in that** the portion of the synthetic in the material is approximately 30 to 70 % by weight.

20. A method for producing a material according to any of Claims 1 to 19, **characterised in that** a carrier made at least partially in the form of a leno fabric having two warp threads intersecting between weft threads running next to one another, said leno fabric having at least one weft thread having a high strength yarn and having warp threads made of an elastomeric material, is coated and/or impregnated with a curable synthetic.

## Revendications

1. Matériau destiné à produire une bande de maintien comportant un support qui est recouvert et/ou imprégné d'une matière plastique durcissable et qui se présente au moins partiellement sous la forme d'un tissu de gaze comportant deux fils de chaîne (12, 14) qui se croisent entre des fils de trame (22, 24) s'étendant côte à côte, le tissu de gaze présentant au moins un fil de trame (22) comportant un fil hautement résistant, notamment un fil de polyester,
**caractérisé en ce que** le tissu présente des fils de chaîne composés d'un matériau élastomère.

2. Matériau selon la revendication 1, **caractérisé en ce que** le tissu présente des fils de chaîne et/ou des fils de trame thermorétractables.

3. Matériau selon l'une des revendications précédentes, **caractérisé en ce que** le matériau présente, dans la direction des fils de chaîne (22, 24, 30) du tissu, préalablement au durcissement de la matière plastique, une extensibilité d'au moins 20 %, de préférence d'au moins 30 %, et plus préférablement d'environ 65 à 95 %.

4. Matériau selon l'une des revendications précédentes, **caractérisé en ce que** le matériau présente, dans la direction des fils de chaîne (22, 24, 30) du tissu et à l'état non étiré, une résistance à la déchirure d'au moins 10 N/cm, de préférence d'au moins 20 N/cm, et plus préférablement d'environ 30 à 60 N/cm.

5. Matériau selon l'une des revendications précédentes, **caractérisé en ce que** le matériau présente, dans la direction des fils de trame (22, 24) du tissu, préalablement au durcissement de la matière plastique, une extensibilité d'au moins 30 %, de préférence d'au moins 40 %, et plus préférablement d'environ 50 à 100 %.

6. Matériau selon l'une des revendications précédentes, **caractérisé en ce que** le matériau présente, dans la direction des fils de trame (22, 24) du tissu et à l'état non étiré, une résistance à la déchirure d'au moins 10 N/cm, de préférence d'au moins 20 N/cm, et plus préférablement d'environ 30 à 50 N/cm.

7. Matériau selon l'une des revendications précédentes, **caractérisé en ce que** le tissu présente, à l'état non étiré, un grammage d'environ 50 à 250 g/cm², de préférence d'environ 100 à 200 g_{/}m².

8. Matériau selon l'une des revendications précédentes, **caractérisé en ce que** les fils de trame et/ou de chaîne (12, 14, 22, 24, 30) du tissu comportent des fibres naturelles.

9. Matériau selon l'une des revendications précédentes, **caractérisé en ce que** les fils de trame et/ou de chaîne (12, 14, 22, 24, 30) du tissu comportent des fibres artificielles telles que des fibres de polyamide, de polyester, de polypropylène ou similaires.

10. Matériau selon l'une des revendications précédentes, **caractérisé en ce que** les fils de trame et/ou de chaîne (12, 14, 22, 24, 30) sont au moins partiellement texturisés pour permettre leur extensibilité.

11. Matériau selon l'une des revendications précédentes, **caractérisé en ce que**, pour au moins une paire de fils de chaîne qui se croisent, l'un des fils de chaîne (12) qui se croisent passe par-dessous les fils de trame (22, 24), l'autre des fils de chaîne (14) qui se croisent passe pardessus les fils de trame (22, 24) et le fil de chaîne (12) passant sous les fils de trame (22, 24) est situé, au niveau des points de croisement (16), au-dessus du fil de chaîne (14) passant au-dessus des fils de trame (22, 24).

12. Matériau selon l'une des revendications précédentes, **caractérisé en ce qu'**au moins deux fils de trame (22, 24) sont situés entre des points de croisement (16) consécutifs des fils de chaîne (12, 14) et/ou des transitions consécutives d'au moins un fil de chaîne supplémentaire.

13. Matériau selon la revendication 12, **caractérisé en ce qu'**un des fils de trame (22, 24) situés entre des points de croisement (16) consécutifs et/ou des transitions consécutives est texturisé et un autre de ces fils de trame (22, 24) consiste en un fil hautement résistant, de préférence lisse, notamment un fil de polyester.

14. Matériau selon l'une des revendications précédentes, **caractérisé en ce que** les fils de trame (22, 24) se présentent sous la forme de segments de fil parmi lesquels au moins deux sont reliés l'un à l'autre par le biais d'un segment de liaison situé sur le bord du tissu.

15. Matériau selon la revendication 14, **caractérisé en ce qu'**au moins un segment de liaison reliant l'un à l'autre deux fils de trame est enroulé autour d'au moins un et de préférence d'au moins deux fils de trame précédents.

16. Matériau selon l'une des revendications précédentes, **caractérisé en ce que** le tissu présente, à l'état non étiré, d'environ 40 à 80, de préférence 66 fils de chaîne et/ou d'environ 80 à 150, de préférence 132 fils de trame pour une longueur, voire une largeur, de tissu de 10 cm.

17. Matériau selon l'une des revendications précédentes, **caractérisé en ce que** la matière plastique est durcissable sous l'effet d'un solvant, notamment de l'eau.

18. Matériau selon la revendication 17, **caractérisé en ce que** la matière plastique comporte un prépolymère polyuréthane durcissable à l'humidité.

19. Matériau selon l'une des revendications précédentes, **caractérisé en ce que** la proportion de matière plastique par rapport au matériau est d'environ 30 à 70 % en poids.

20. Procédé de production d'un matériau selon l'une des revendications 1 à 19, **caractérisé en ce qu'**un support, qui se présente au moins partiellement sous la forme d'un tissu de gaze comportant deux fils de chaîne qui se croisent entre des fils de trame s'étendant côte à côte, le tissu de gaze présentant au moins un fil de trame comportant un fil hautement résistant et présentant des fils de chaîne composés d'un matériau élastomère, est recouvert et/ou imprégné d'une matière plastique durcissable.
